# EUROPEAN PATENT APPLICATION

(11) **EP 4 047 609 A1**
(43) Date of publication of application: **24.08.2022**
(21) Application number: 20876386.2
(22) Date of filing: 05.10.2020
(51) Int. Cl.: G16H 10/00, G16H 50/20

(54) **INFORMATION PROCESSING DEVICE, INFORMATION PROCESSING METHOD, AND PROGRAM**

(30) Priority: 15.10.2019 JP 2019188702
(71) Applicant: Sony Group Corporation, Tokyo 108-0075 (JP)
(72) Inventor: KATSU Masanori, Tokyo 108-0075 (JP); SHIGYO Maki, Tokyo 108-0075 (JP); WAKITA Yoshihiro, Tokyo 108-0075 (JP)
(74) Representative: MFG Patentanwälte Meyer-Wildhagen Meggle-Freund Gerhard PartG mbB
(86) International application number: PCT/JP2020/037693
(87) International publication number: WO 2021/075289

(57) **Abstract**

The present technology relates to an information processing apparatus, an information processing method, and a program that enable effective utilization of data in a non-medical device.

There is provided an information processing apparatus including a processing unit that makes a determination regarding a user as a measurement target according to intended use by using a determination model that has learned with machine learning using first measurement data measured by a medical device and second measurement data measured by a non-medical device so as to suit intended use. The present technology can be applied to, for example, a system that processes measurement data from a medical device and a non-medical device.

## Description

### TECHNICAL FIELD

The present technology relates to an information processing apparatus, an information processing method, and a program, and more particularly to an information processing apparatus, an information processing method, and a program that enable effective utilization of data in a non-medical device.

### BACKGROUND ART

In recent years, a device equipped with a vital sign sensor that detects a vital sign of a user has become widespread as a device such as a wearable terminal.

Furthermore, as a technique using a vital sign sensor, for example, a technique disclosed in Patent Document 1 is known. In Patent Document 1, weighting is performed when biological information sensed by a plurality of sensors is integrated, and the integrated biological information is output.

### CITATION LIST

### PATENT DOCUMENT

Patent Document 1: Japanese Patent Application Laid-Open No. 2018-33771

### SUMMARY OF THE INVENTION

### PROBLEMS TO BE SOLVED BY THE INVENTION

Incidentally, it is assumed that data measured by a non-medical device equipped with a vital sign sensor will be utilized for medical care along with data measured by a medical device. Therefore, it is required to effectively utilize the data in the non-medical device.

The present technology has been developed in view of the above circumstances, and is to enable effective utilization of data in a non-medical device.

### SOLUTIONS TO PROBLEMS

An information processing apparatus according to one aspect of the present technology includes a processing unit that makes a determination regarding a user as a measurement target according to intended use by using a determination model that has learned with machine learning using first measurement data measured by a medical device and second measurement data measured by a non-medical device so as to suit intended use.

An information processing method according to one aspect of the present technology includes, by the information processing apparatus, making a determination regarding a user as a measurement target according to intended use by using a determination model that has learned with machine learning using first measurement data measured by a medical device and second measurement data measured by a non-medical device so as to suit intended use.

In the information processing apparatus and the information processing method according to one aspect of the present technology, a determination regarding a user as a measurement target is made according to intended use by using a determination model that has learned with machine learning using first measurement data measured by a medical device and second measurement data measured by a non-medical device so as to suit intended use.

The information processing apparatus according to one aspect of the present technology includes a processing unit that learns, at a time of generation of a determination model for making a determination regarding a user as a measurement target according to intended use, with machine learning using first measurement data measured by a medical device and second measurement data measured by a non-medical device so as to suit intended use.

The information processing method according to one aspect of the present technology includes, by the information processing apparatus, learning, at a time of generation of a determination model for making a determination regarding a user as a measurement target according to intended use, with machine learning using first measurement data measured by a medical device and second measurement data measured by a non-medical device so as to suit intended use.

A program according to one aspect of the present technology causes a computer to function as a processing unit that learns, at a time of generation of a determination model for making a determination regarding a user as a measurement target according to intended use, with machine learning using first measurement data measured by a medical device and second measurement data measured by a non-medical device so as to suit intended use.

In the information processing apparatus, the information processing method, and the program according to one aspect of the present technology, learning is performed, at a time of generation of a determination model for making a determination regarding a user as a measurement target according to intended use, with machine learning using first measurement data measured by a medical device and second measurement data measured by a non-medical device so as to suit intended use.

The information processing apparatus according to one aspect of the present technology may be an independent apparatus or may be an inner block including one apparatus.

### BRIEF DESCRIPTION OF DRAWINGS

Fig. 1 is a diagram illustrating an example of a configuration of an embodiment of an information processing system to which the present technology is applied.
Fig. 2 is a diagram illustrating an example of a configuration of an embodiment of an information processing apparatus to which the present technology is applied.
Fig. 3 is a diagram illustrating an example of a configuration of an embodiment of the information processing apparatus to which the present technology is applied.
Fig. 4 is a diagram illustrating an example of an observation screen.
Fig. 5 is a flowchart describing a flow of observation screen generation processing.
Fig. 6 is a diagram illustrating an example of learning of a determination model for daily guideline.
Fig. 7 is a diagram illustrating an example of learning of a determination model for medical intervention.
Fig. 8 is a diagram illustrating an example of learning of a determination model at a time of approval or revocation as a medical device.
Fig. 9 is a diagram illustrating an example of a configuration of a database.
Fig. 10 is a diagram illustrating an example of an integration DB.
Fig. 11 is a diagram illustrating an example of an approved device DB.
Fig. 12 is a diagram illustrating an example of a learning DB.
Fig. 13 is a diagram illustrating another example of the integration DB.
Fig. 14 is a diagram illustrating another example of a configuration of a server.
Fig. 15 is a flowchart describing a flow of processing at a time of learning.
Fig. 16 is a flowchart describing a flow of processing at a time of determination.
Fig. 17 is a diagram illustrating an example of a determination result screen.
Fig. 18 is a flowchart describing a flow of determination result screen generation processing.
Fig. 19 is a diagram illustrating an example of a medical interview support tool for patient.
Fig. 20 is a diagram illustrating an example of the medical interview support tool for patient.
Fig. 21 is a diagram illustrating a first example of differentiation display of the medical device and a non-medical device.
Fig. 22 is a diagram illustrating a second example of differentiation display of the medical device and the non-medical device.
Fig. 23 is a diagram illustrating a configuration example of a computer.

### MODE FOR CARRYING OUT THE INVENTION

Hereinafter, an embodiment of the present technology will be described with reference to the drawings. Note that the description will be made in the following order.

1. Embodiment of the present technology
2. Modifications
3. Configuration of computer

### <1. Embodiment of the present technology>

### (Configuration of system)

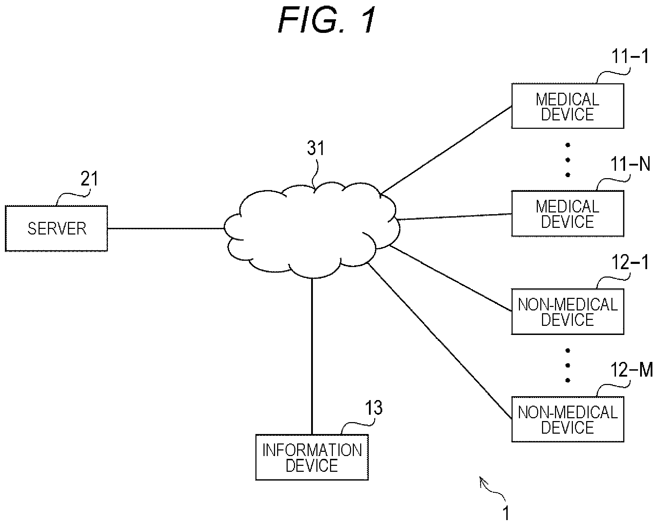

Fig. 1 illustrates an example of a configuration of an embodiment of an information processing system to which the present technology is applied.

In Fig. 1, an information processing system 1 includes medical devices 11-1 to 11-N (N: an integer of 1 or more), non-medical devices 12-1 to 12-M (M: an integer of 1 or more), an information device 13, and a server 21.

In the information processing system 1, the medical devices 11-1 to 11-N, the non-medical devices 12-1 to 12-M, the information device 13, and the server 21 are mutually connected via a network 31. The network 31 includes a communication network such as the Internet, an intranet, or a mobile telephone network.

The medical device 11-1 is a medical device such as a portable electrocardiograph. The medical device 11-1 measures a heart rate, electrocardiogram waveform, or the like of a user (patient) with a vital sign sensor or the like, and transmits measurement data to the server 21 via the network 31.

The medical devices 11-2 to 11-N are various medical devices that measure data and transmit the measurement data to the server 21 via the network 31.

The non-medical device 12-1 is a non-medical device such as a wristwatch-like wearable terminal or smartphone, and is equipped with a vital sign sensor that detects a vital sign (blood pressure, a pulse, a respiratory rate, body temperature, or the like) of the user (patient). The non-medical device 12-1 measures a heart rate, sleeping hours, or the like of the user, and transmits the measurement data to the server 21 via the network 31.

The non-medical devices 12-2 to 12-M are various non-medical devices that measure data and transmit the measurement data to the server 21 via the network 31.

Here, the "medical device" is a device intended to be used for diagnosis, treatment, or prevention of diseases in human, or the like, and is regulated under "Act on Securing Quality, Efficacy and Safety of Products Including Pharmaceuticals and Medical Devices (abbreviated name: Pharmaceutical and Medical Device Act)" in Japan.

In the present description, a device that does not fall under the definition of a medical device is referred to as a "non-medical device". Furthermore, a medical device and a non-medical device are collectively referred to as a "measurement device".

However, definitions of "medical device" and "non-medical device" are not limited thereto. For example, it is assumed that a stipulation will be made for a "quasi-medical device" according to Pharmaceutical and Medical Device Act, in which a "quasi-medical device" is treated as a non-medical device in a case where the device is used by a user individually, and as a medical device in a case where the device is used under responsibility of a physician, or the like.

For example, although there are some non-medical devices that cannot be treated as medical devices due to having poor measurement accuracy, a non-medical device as a quasi-medical device can be treated as a medical device in a case where the non-medical device is used under responsibility of a physician. In the present description, such a quasi-medical device under management of a physician can be included in the medical device.

Note that, in the following description, the medical devices 11-1 to 11-N will also be simply referred to as a medical device 11 in a case where the devices are not particularly necessary to be differentiated from one another. Furthermore, the non-medical devices 12-1 to 12-M will also be simply referred to as a non-medical device 12 in a case where the devices are not particularly necessary to be differentiated from one another.

Furthermore, measurement data measured by the medical device 11 is referred to as "medical device measurement data", while measurement data measured by the non-medical device 12 is referred to as "non-medical device measurement data". Moreover, the medical device measurement data and the non-medical device measurement data are collectively referred to as "measurement data".

The information device 13 is a device such as a smartphone, a tablet terminal, a personal computer, or a display apparatus. The information device 13 is used by a user (mainly a physician) and performs various operations corresponding to operation by the physician or the like.

For example, the information device 13 transmits a request to the server 21 to receive transmission data transmitted from the server 21 via the network 31. The information device 13 presents various pieces of information on the basis of the received transmission data.

Via the network 31, the server 21 receives medical device measurement data transmitted from the medical device 11 and non-medical device measurement data transmitted from the non-medical device 12. The server 21 stores and manages the received measurement data in a database.

Furthermore, the server 21 executes various processing on the basis of the measurement data stored in the database. For example, in a case where the server 21 receives a request from the information device 13, the server 21 generates transmission data on the basis of the measurement data and transmits the transmission data to the information device 13 via the network 31.

Note that, although a case where the medical device 11 and the non-medical device 12 are equipped with a vital sign sensor is described above, the medical device 11 and the non-medical device 12 may be equipped with a biomedical sensor including, for example, a blood pressure sensor, a blood oxygen level sensor, a respiration sensor, a moisture sensor, a temperature sensor, an electrocardiogram sensor or the like, or a motion sensor, an image sensor, an inertial sensor, a pressure sensor, a bed leaving sensor, or the like, and may use measurement data measured by the biomedical sensor.

### (Configuration of information device)

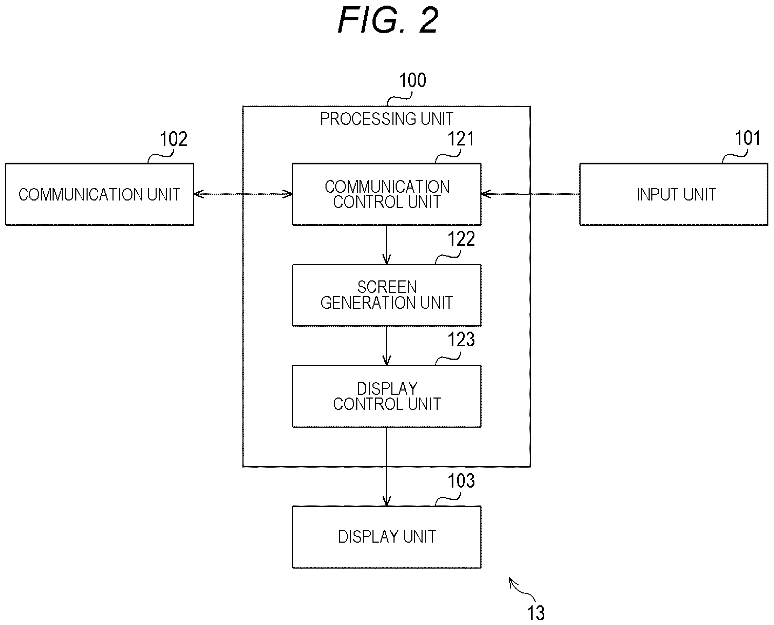

Fig. 2 illustrates an example of a configuration of the information device 13 in Fig. 1 as an example of a configuration of an embodiment of an information processing apparatus to which the present technology is applied.

In Fig. 2, the information device 13 includes a processing unit 100, an input unit 101, a communication unit 102, and a display unit 103.

The processing unit 100 is a central processing apparatus (control apparatus) that controls operation of each unit and performs various arithmetic processing, and includes a processor such as a central processing unit (CPU). The processing unit 100 controls operation of each of the units in the information device 13.

The input unit 101 is an input apparatus such as a physical button or a touch panel. The input unit 101 supplies the processing unit 100 with an operation signal corresponding to operation by the physician or the like. The processing unit 100 performs various processing on the basis of the operation signal supplied from the input unit 101.

The communication unit 102 is a communication apparatus (communication module) that supports wireless communication, such as wireless local area network (LAN) or cellular communication (for example, LTE-Advanced, 5G, or the like), or wired communication. The communication unit 102 communicates with another device via the network 31 under control of the processing unit 100.

The display unit 103 is a display apparatus such as a liquid crystal panel or an organic light emitting diode (OLED) panel. The display unit 103 displays various screens under control of the processing unit 100.

Note that, in a case where the input unit 101 and the display unit 103 constitute a touch panel, when a finger of the physician or the like comes in contact with (touches) a surface of the display unit 103, the input unit 101 supplies the processing unit 100 with an operation signal corresponding to the contact position (position of a predetermined point).

Furthermore, the processing unit 100 includes a communication control unit 121, a screen generation unit 122, and a display control unit 123.

The communication control unit 121 controls the communication unit 102 on the basis of the operation signal from the input unit 101, and exchanges data with another device such as the server 21 via the network 31. For example, the communication control unit 121 receives transmission data transmitted from the server 21 and supplies the transmission data to the screen generation unit 122.

The screen generation unit 122 generates, on the basis of the transmission data supplied from the communication control unit 121, display data for the various screens to be displayed on the display unit 103, and supplies the display data to the display control unit 123.

The display control unit 123 displays the various screens on the display unit 103 on the basis of the display data supplied from the screen generation unit 122.

Note that the configuration of the information device 13 illustrated in Fig. 2 is merely an example, and, for example, a sensor unit including a motion sensor such as an angular velocity sensor, an acceleration sensor, or a geomagnetic sensor, a biomedical sensor such as a fingerprint sensor or a vein authentication sensor, or a proximity sensor, a camera unit including an image sensor or a signal processing unit, an auxiliary storage unit including a semiconductor memory such as a non-volatile memory, or the like may be provided.

### (Configuration of server)

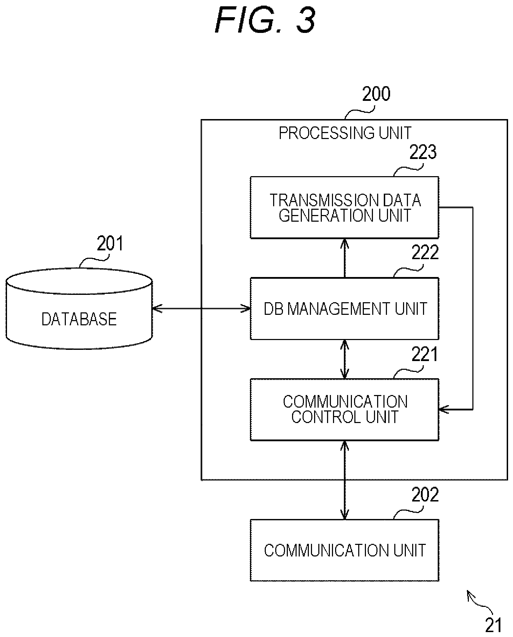

Fig. 3 illustrates an example of a configuration of the server 21 in Fig. 1 as an example of a configuration of an embodiment of the information processing apparatus to which the present technology is applied.

In Fig. 3, the server 21 includes a processing unit 200, a database 201, and a communication unit 202.

The processing unit 200 is a central processing apparatus (control apparatus) that controls operation of each unit and performs various arithmetic processing, and includes a processor such as a CPU. The processing unit 200 controls operation of each of the units in the server 21.

The database 201 is included in an internal storage provided in the server 21 or in an external storage. The database 201 stores various data under control of the processing unit 200.

The communication unit 202 is a communication apparatus that supports wireless communication such as a wireless LAN or wired communication such as Ethernet (registered trademark). The communication unit 202 communicates with another device via the network 31 under control of the processing unit 200.

Furthermore, the processing unit 200 includes a communication control unit 221, a DB management unit 222, and a transmission data generation unit 223.

The communication control unit 221 controls the communication unit 202 and exchanges data with another device such as the medical device 11, the non-medical device 12, or the information device 13 via the network 31.

For example, the communication control unit 221 receives medical device measurement data transmitted from the medical device 11 and non-medical device measurement data transmitted from the non-medical device 12, and supplies the DB management unit 222 with the medical device measurement data and the non-medical device measurement data. Furthermore, the communication control unit 221 transmits the transmission data supplied from the transmission data generation unit 223 to the information device 13.

The DB management unit 222 manages the database 201. For example, the DB management unit 222 stores, in the database 201, the measurement data (medical device measurement data and non-medical device measurement data) supplied from the communication control unit 221. Furthermore, the DB management unit 222 reads the measurement data stored in the database 201 and supplies the measurement data to the transmission data generation unit 223.

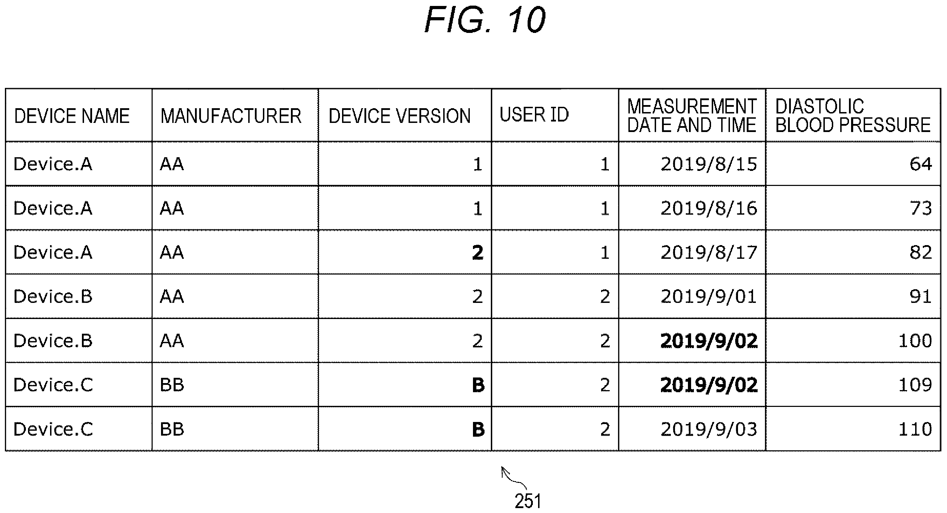

Note that the measurement data can include information such as time information related to measurement (measurement date and time) or identification information of a user as a measurement target (user ID or the like), in addition to a measured value. For example, as the database 201, an integration DB 251 (Fig. 10) to be described later can be used.

The transmission data generation unit 223 generates, on the basis of the measurement data supplied from the DB management unit 222, transmission data to be transmitted to the information device 13, and supplies the transmission data to the communication control unit 221.

### (Display example of observation screen)

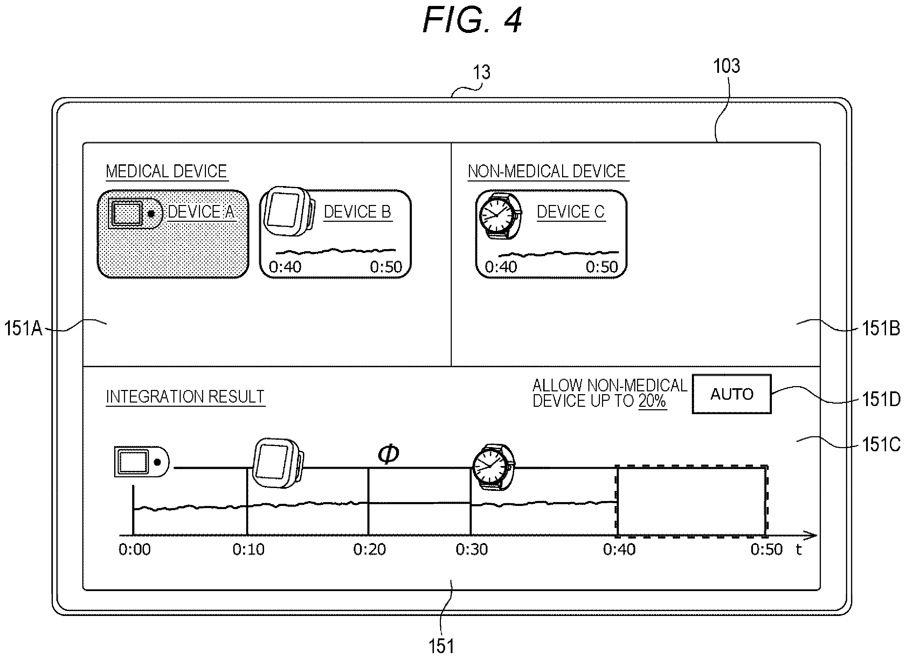

Fig. 4 illustrates an example of an observation screen displayed on the display unit 103 of the information device 13 such as a tablet terminal used by the physician or the like.

In Fig. 4, an observation screen 151 includes three regions in which an entire region of the screen is horizontally divided into two and an upper region thereof is vertically divided into two.

An upper left region 151A displays information regarding the medical device 11. An upper right region 151B displays information regarding the non-medical device 12. That is to say, measurement devices used by the user (patient) are sorted as the medical device 11 or the non-medical device 12, and information (log information or the like) regarding each of the sorted measurement devices is displayed in the upper left region 151A or the upper right region 151B.

In the upper left region 151A, a device A and a device B are sorted as the medical device 11, and a graph (for example, a line graph) based on medical device measurement data in a specified section is displayed for each of the devices.

In this example, the specified section (0:40 to 0:50) is a non-measurement section for the device A because measurement is not performed by the device A during the specified section, and therefore, information regarding the device A is displayed gray out.

Meanwhile, because measurement is performed by the device B during the specified section (0:40 to 0:50), a graph or the like illustrating medical device measurement data in time series for the specified section is displayed as information regarding the device B. At this time, an image (still image or icon) of the device B is displayed, and therefore, the physician or the like can intuitively recognize that medical device measurement data in the device B has been used.

In the upper right region 151B, a device C is sorted as the non-medical device 12, and a graph or the like illustrating non-medical device measurement data in time series for the specified section (0:40 to 0:50) is displayed as information regarding the device C. At this time, display of an image of the device C clearly indicates that non-medical device measurement data in the device C has been used.

Note that, although information (log information or the like) regarding these measurement devices is automatically arranged when the observation screen 151 is displayed, the physician or the like may manually arrange information regarding measurement data in each of the measurement devices. For example, with respect to a desired section, the physician may arrange information regarding a desired measurement device or discard information regarding an arranged measurement device with drag-and-drop operation.

A lower region 151C displays an integration result obtained by integrating information regarding the medical device 11 and information regarding the non-medical device 12. As the integration result, a graph based on the medical device measurement data or on the non-medical device measurement data is displayed for each specified section, according to a proportion of the non-medical device 12 in all the measurement devices, or the like.

In the lower region 151C, the device A as a medical device 11 is decided to be used in a first section (0:00 to 0:10), and a graph based on medical device measurement data in the device A is displayed. In the first section, an image (still image or icon) of the device A is displayed, and therefore, the physician or the like can intuitively recognize that medical device measurement data in the device A has been used.

The device B as a medical device 11 is decided to be used in a second section (0:10 to 0:20), and a graph based on medical device measurement data in the device B is displayed. In the second section, display of an image of the device B clearly indicates that medical device measurement data in the device B has been used.

In a third section (0:20 to 0:30), measurement is not performed by all the measurement devices, and therefore, a graph interpolated by using measurement data in the previous and next sections (0:10 to 0:20, 0:30 to 0:40) is displayed. Note that, although the case where the interpolated data is displayed is exemplified here, another display form such as hiding the section may be used.

The device C as the non-medical device 12 is decided to be used in a fourth section (0:30 to 0:40), and a graph based on non-medical device measurement data in the device C is displayed. In the fourth section, display of an image of the device C clearly indicates that non-medical device measurement data in the device C has been used.

A fifth section (0:40 to 0:50) represents, with highlight display represented by a broken line in the drawing, a section to be selected by a physician or the like. For example, with respect to the fifth section, the physician or the like can manually arrange information regarding measurement data in a desired measurement device.

An automatic arrangement button 151D is a button for a physician or the like to instruct automatic arrangement of the integration result. With the automatic arrangement, it is possible to specify how much a proportion of the non-medical device 12 in all the measurement devices is allowed. In this example, in a case where the automatic arrangement button 151D is operated by being tapped by the physician or the like, the integration result is redisplayed in a state where the proportion of the non-medical device 12 is allowed up to 20%.

Note that, although, in the above description, images of the measurement devices are displayed along with graphs for each section to clearly indicate which measurement device the information is displayed regarding, a character string (text), such as "medical device" or "non-medical device" for example, may be displayed other than a still image, an icon, or the like. Furthermore, the specified section may be not only specified by the physician or the like as a matter of course, but also set by a system side.

### (Flow of generation of observation screen)

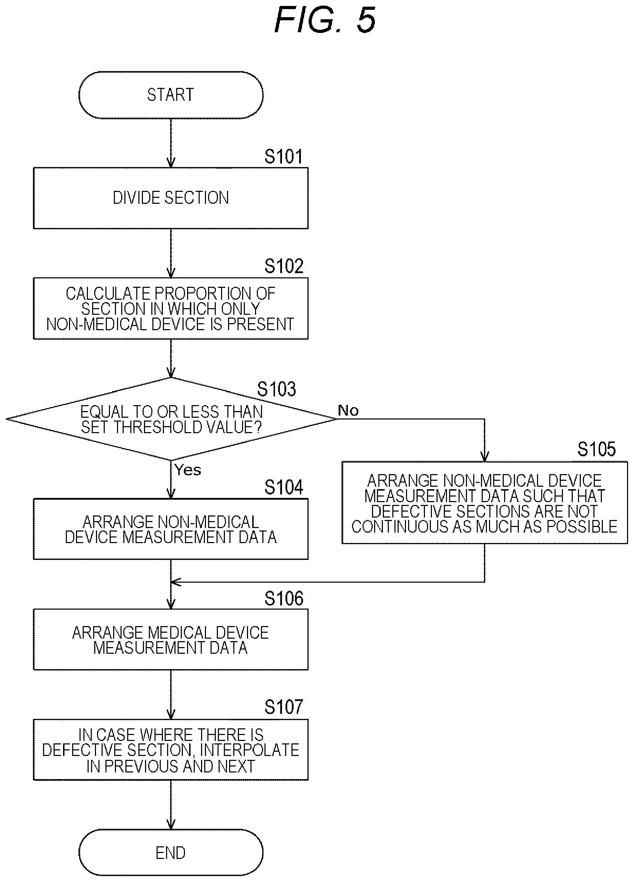

Next, a flow of observation screen generation processing will be described with reference to the flowchart in Fig. 5.

The observation screen generation processing is executed by the processing unit 100 (screen generation unit 122 or the like) of the information device 13, or is executed by the processing unit 200 (transmission data generation unit 223 or the like) of the server 21. Here, description will be given assuming that the processing unit 200 executes the observation screen generation processing.

In Step S101, the transmission data generation unit 223 divides the section in time series in which measurement data is arranged when arranging the integration result.

In Step S102, the transmission data generation unit 223 calculates a proportion of a divided section in which only the non-medical device 12 is present with reference to measurement data of a target user, the measurement data being stored in the database 201.

In Step S103, the transmission data generation unit 223 determines whether or not the calculated proportion is equal to or less than a set threshold value.

In a case where it is determined in the determination processing in Step S103 that the calculated proportion is equal to or less than the set threshold value, the processing proceeds to Step S104. In Step S104, the transmission data generation unit 223 arranges non-medical device measurement data read from the database 201 in the target section. With this arrangement, non-medical device measurement data is arranged in the section in which only non-medical device measurement data is present.

Meanwhile, in a case where it is determined in the determination processing in Step S103 that the calculated proportion exceeds the set threshold value, the processing proceeds to Step S105. In Step S105, the transmission data generation unit 223 arranges non-medical device measurement data read from the database 201 in the target section such that defective sections are not continuous as much as possible.

When the processing in Step S104 or S105 ends, the processing proceeds to Step S106.

In Step S106, the transmission data generation unit 223 arranges medical device measurement data read from the database 201 in the target section.

At this time, in a case where there is a plurality of pieces of medical device measurement data or non-medical device measurement data in the same section, it is only required to decide which measurement data is to be used on the basis that the measurement data occupies a larger proportion of the section (log rate is high), has small variance, or the like.

In Step S107, in a case where there is a defective section, the transmission data generation unit 223 interpolates the section by using measurement data in previous and next sections.

With this arrangement, an integration result to be displayed in the lower region 151C of the observation screen 151 (Fig. 4) is generated. Furthermore, information regarding the medical device 11 displayed in the upper left region 151A is generated on the basis of medical device measurement data read from the database 201, and information regarding the non-medical device 12 displayed in the upper right region 151B is generated on the basis of non-medical device measurement data read from the database 201.

The integration result generated in this way and the transmission data including the information regarding the medical device 11 and non-medical device 12 are transmitted to the information device 13 via the network 31. Then, in the information device 13, the processing unit 100 displays the observation screen 151 (Fig. 4) on the display unit 103 on the basis of the transmission data received from the server 21.

Note that, in a case where the processing unit 100 of the information device 13 executes the observation screen generation processing, the server 21 is only required to transmit, as transmission data, the medical device measurement data and the non-medical device measurement data stored in the database 201 to the information device 13 via the network 31.

The flow of the observation screen generation processing has been described above. In the observation screen generation processing, the observation screen 151 using not only medical device measurement data but also non-medical device measurement data as measurement data is generated, and therefore, the non-medical device measurement data can be effectively utilized. Furthermore, the physician can display the observation screen 151 on the information device 13 at a time of diagnosis and observe the integration result or the like. Furthermore, the physician can also hide information regarding the non-medical device 12 as necessary.

### (Learning of determination model)

Incidentally, in recent years, wearable terminals or the like equipped with a vital sign sensor have become widespread, and there has been a tendency to utilize measurement data measured by a vital sign sensor for medical care.

However, non-medical device measurement data measured by a non-medical device 12, such as a wearable terminal, must not be overestimated and should not be used for a certain judgment by a physician. In Japan, this is stipulated by Pharmaceutical and Medical Device Act.

That is, although it is not a problem to use information regarding non-medical device measurement data as a reference on risk judgment, it may cause malpractice or the like to make a safety judgment just because a measured value is normal, for example.

Meanwhile, with devices equipped with a vital sign sensor such as a wearable terminal becoming widespread, a uniform prohibition of utilizing non-medical device measurement data for medical care, the prohibition focusing on only a negative side only, may lead to not utilizing daily accumulated data, and therefore, it is required to effectively utilize the non-medical device measurement data.

In the present technology, a determination model that has learned with machine learning using medical device measurement data and non-medical device measurement data so as to suit intended use is generated, and a determination is made according to the intended use by using the determination model.

As a machine learning method, for example, a neural network or deep learning is used.

A neural network is a model imitating a neural circuit of a human brain, and includes three types of layers that are an input layer, an intermediate layer (hidden layer), and an output layer. Furthermore, deep learning is a model using a neural network having a multi-layer structure, and is capable of learning a complex pattern hidden in a large amount of data by repeating characteristic learning in each of the layers.

Furthermore, a problem setting in machine learning includes supervised learning, unsupervised learning, semi-supervised learning, reinforcement learning, inverse reinforcement learning, active learning, transfer learning, and the like.

For example, in the supervised learning, a feature amount is learned on the basis of given labeled learning data (supervised data). With this arrangement, it is possible to derive a label for unknown data. Furthermore, In the unsupervised learning, a large amount of unlabeled learning data is analyzed to extract a feature amount, and clustering is performed on the basis of the extracted feature amount. With this arrangement, it is possible to analyze a trend and predict future on the basis of a huge amount of unknown data.

As a determination model, according to intended use, there are a determination model for daily guideline and a determination model for medical intervention. The determination model for daily guideline is a model for determining a daily guideline (a guideline without medical intervention) for a user. The determination model for medical intervention is a model for making a determination when medical intervention is performed on a user.

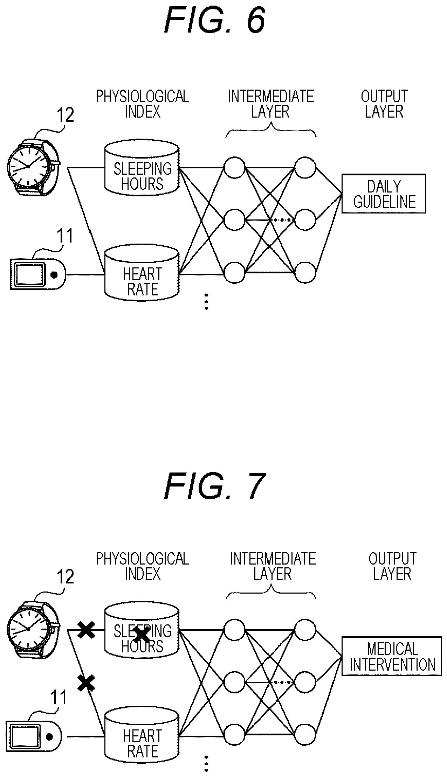

Fig. 6 illustrates an example of learning of a determination model for daily guideline.

Figure 6 illustrates an example of machine learning using a neural network. The neural network has a physiological index as the input layer, the intermediate layer, and the output layer, and each of the layers has a structure in which a plurality of nodes is connected by edges. Note that the white circles (∘) in the drawing represent the nodes, and the straight lines connecting each of the white circles represent the edges.

The intermediate layer can have a plurality of layers, and the one having a particularly deep intermediate layer is referred to as deep learning. Here, each of the layers has a function referred to as an activation function, and the edges can have a weight. Then, a value of each of the nodes is calculated from a value of a node in a previous layer connecting to the node, that is to say, the value of the node in the previous layer, a value of weight of a connection edge, and an activation function that belongs to a layer.

At a time of learning of a determination model for daily guideline, non-medical device measurement data (measurement data of a heart rate, sleeping hours, or the like) measured by the non-medical device 12 is input along with medical device measurement data (measurement data of a heart rate or the like) measured by the medical device 11, by which learning using the measurement data is performed in the intermediate layer, and the determination model for daily guideline is generated.

At this time, if the same index can be calculated from a plurality of measurement devices, weight is optimized by learning (accuracy of measurement data, or the like). For example, in a case where a user rarely uses a measurement device manufactured by a certain manufacturer, it is only required to change weight of the measurement device to reduce the weight.

Thus, when a daily guideline is determined, a judgment by the physician may include the non-medical device measurement data measured by the non-medical device 12, and therefore, at a time of learning of a determination model for daily guideline, the non-medical device measurement data is input along with the medical device measurement data.

Fig. 7 illustrates an example of learning of a determination model for medical intervention.

Similarly to Figure 6, Fig. 7 illustrates an example of machine learning using a neural network. At a time of learning of a determination model for medical intervention, medical device measurement data (measurement data of a heart rate or the like) measured by the medical device 11 is input, by which learning using the measurement data is performed in the intermediate layer, and the determination model for medical intervention is generated.

That is to say, when a medical intervention is determined, there is a restriction that a judgment by the physician must not include the non-medical device measurement data measured by the non-medical device 12, and therefore, only the medical device measurement data is input. At this time, weight of (the non-medical device measurement data of) the non-medical device 12 is changed to 0.

Because the determination model for medical intervention is generated in this way, when a medical intervention is determined for a certain user, a determination result with higher performance can be obtained by using the determination model for medical intervention than by making estimation for the determination model for daily guideline by using measurement data obtained by excluding non-medical device measurement data from the measurement data of the user.

Here, the measurement data used as learning data is collected without differentiating the medical device measurement data and the non-medical device measurement data at a time of the collection from the measurement devices, and is accumulated as the same data set. Then, at a time of learning, the learning DB is generated by differentiating the medical device measurement data and the non-medical device measurement data, and the learning processing using the generated learning DB is performed.

With this arrangement, even in a case where a certain non-medical device is approved as a medical device at a certain time point, measurement data collected when the device was the non-medical device can be utilized at a time of learning after the device becomes a medical device. Therefore, the collected measurement data can be utilized without being wasted.

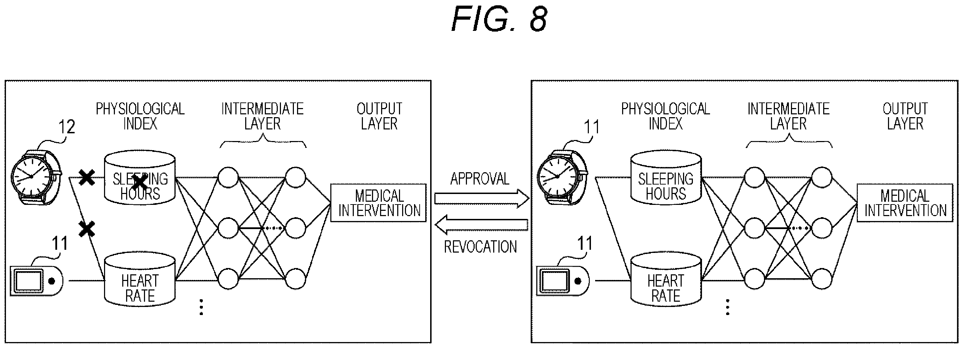

Fig. 8 illustrates an example of learning of a determination model at a time of approval or revocation as the medical device.

Fig. 8 illustrates an example of learning of a determination model for medical intervention in a case where a wristwatch-type wearable terminal, which used to be the non-medical device, is changed from the non-medical device 12 to the medical device 11 by being approved as a medical device.

When the wristwatch-type wearable terminal is the non-medical device 12 before the approval in the left frame in the drawing, a judgment by the physician must not be based on the non-medical device measurement data, and therefore, a determination model for medical intervention is generated by only the medical device measurement data (measurement data of a heart rate or the like) measured by the medical device 11 being input, provided that weight of the wristwatch-type wearable terminal is set to 0.

Meanwhile, when the wristwatch-type wearable terminal becomes the medical device 11 after the approval in the right frame in the drawing, medical device measurement data (measurement data of a heart rate, sleeping hours, or the like) measured by the wristwatch-type wearable terminal is input along with the medical device measurement data (measurement data of a heart rate or the like) measured by the medical device 11, and a determination model for medical intervention is generated.

Note that, although a case where the wristwatch-type wearable terminal is approved as a medical device has been described above, conversely, in a case where the approval of the wristwatch-type wearable terminal as a medical device has been revoked, the wristwatch-type wearable terminal is only required to be treated not as the medical device 11, but as the non-medical device 12. That is, at this time, the measurement data measured by the wristwatch-type wearable terminal is treated as non-medical device measurement data at a time of learning.

### (Example of database)

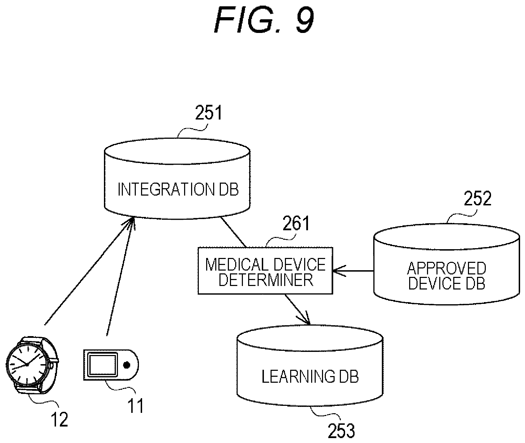

Fig. 9 is a diagram illustrating an example of a configuration of the database 201.

In the server 21, the database 201 can include the integration DB 251, an approved device DB 252, and a learning DB 253.

The integration DB 251 stores the medical device measurement data transmitted from the medical device 11 and non-medical device measurement data transmitted from the non-medical device 12.

The approved device DB 252 stores information regarding the medical device 11 approved as a medical device.

When reading the measurement data stored in the integration DB 251, a medical device determiner 261 refers to information stored in the approved device DB 252 to read the medical device measurement data and the non-medical device measurement data, or read the medical device measurement data, and then stores the data in the learning DB 253 as learning data.

The learning DB 253 stores information regarding learning data used at a time of learning. For example, the learning DB 253 stores the medical device measurement data and the non-medical device measurement data as learning data at a time of learning of a determination model for daily guideline. Furthermore, the learning DB 253 stores the medical device measurement data as learning data at a time of learning of the determination model for medical intervention.

Fig. 10 illustrates an example of the integration DB 251.

The table illustrated in Fig. 10 stores a plurality of records corresponding to the measurement data in a predetermined order, and stores information corresponding to each of column names, which are DEVICE NAME, MANUFACTURER, DEVICE VERSION, USER ID, MEASUREMENT DATE AND TIME, and DIASTOLIC BLOOD PRESSURE for each column with the column names.

The columns with the column names that are DEVICE NAME, MANUFACTURER, and DEVICE VERSION store measurement device information. Furthermore, the columns with the column names that are USER ID, MEASUREMENT DATE AND TIME, and DIASTOLIC BLOOD PRESSURE store vital sign information regarding a vital sign detected by the vital sign sensor. That is to say, the measurement data includes measurement device information and vital sign information. Furthermore, in this example, the vital sign is diastolic blood pressure detected by the vital sign sensors, and the vital sign information is information including a user ID, measurement date and time, and diastolic blood pressure.

The DEVICE NAME column stores information regarding a name of the measurement device. The MANUFACTURER column stores information regarding a manufacturer manufactured the measurement device. The DEVICE VERSION column stores information regarding a version of the measurement device. By managing a device version, it is possible to support a version upgrade after purchasing the measurement device.

The USER ID column stores identification information (user ID or the like) of the user as the measurement target. The MEASUREMENT DATE AND TIME column stores time information regarding measurement (measurement date and time, or the like). The DIASTOLIC BLOOD PRESSURE column stores information regarding diastolic blood pressure as a measured value measured by the measurement device.

Specifically, in the table illustrated in Fig. 10, measurement data for three records is stored for the user with User ID "1" during three days from August 15 to August 17, 2019. Furthermore, measurement data for four records is stored for the user with User ID "2" during three days from September 1 to September 3, 2019. Thus, in the table illustrated in Fig. 10, a plurality of pieces of log information regarding the same user of the same day can be stored.

Note that, although the table in Fig. 10 illustrates a case where measurement device information and vital sign information are managed on the same table, the measurement device information and the vital sign information may be linked with each other with a measurement condition ID, or the like and be managed on separate tables.

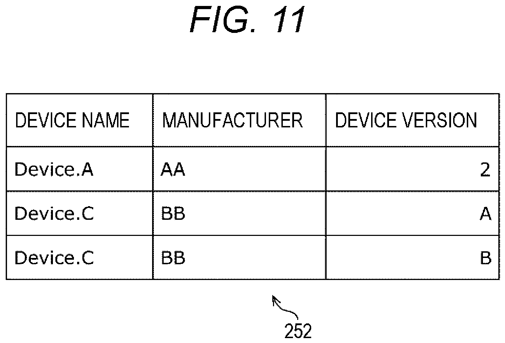

Furthermore, for the device version, an arbitrary character string can be set for each manufacturer, and in that case, it is only required to be able to identify the medical device 11 in combination with the approved device DB 252 (Fig. 11). In this regard, version information may be managed by, for example, defining a standard to include a specific character string for a device version of the medical device 11, or the like, so that all the manufacturers conform to the unified standard.

Fig. 11 illustrates an example of the approved device DB 252.

The table illustrated in Fig. 11 stores information corresponding to the column names in respective columns with the column names that are DEVICE NAME, MANUFACTURER, and DEVICE VERSION.

The column names in the table of the approved device DB 252 correspond to some column names in the table of the integration DB 251 in Fig. 10, and information for three records is stored as information regarding the approved measurement device (medical device 11). That is to say, this example illustrates a case where an arbitrary character string is used for a device version for each manufacturer.

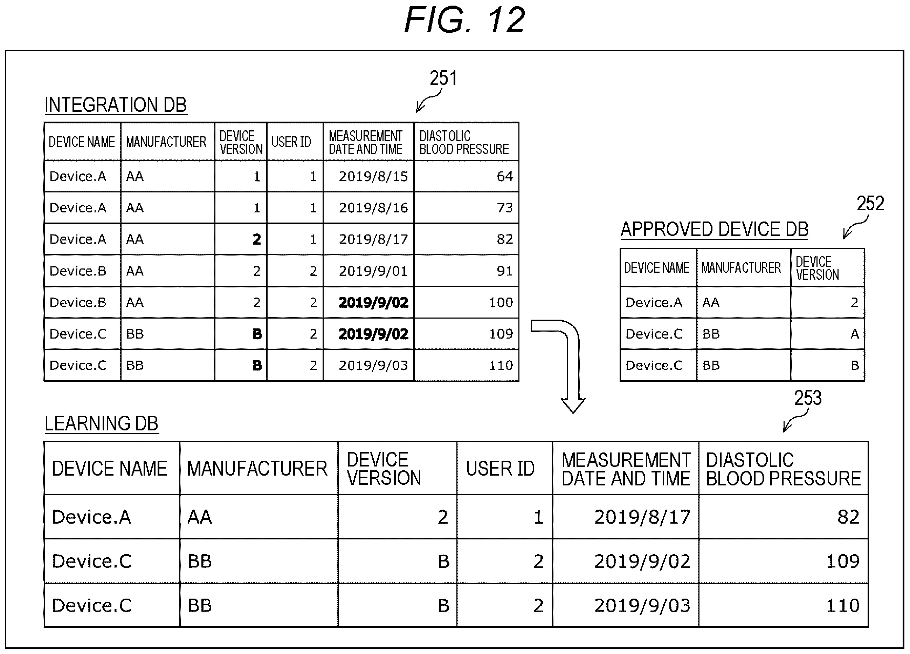

Fig. 12 illustrates an example of the learning DB 253.

Note that Fig. 12 illustrates the integration DB 251 (Fig. 10) and the approved device DB 252 (Fig. 11) for sake of clarity.

The learning DB 253 is generated by the medical device determiner 261 (Fig. 9) reading the measurement data stored in the integration DB 251 on the basis of the information stored in the approved device DB 252.

For example, because, at a time of learning of the determination model for medical intervention, it is necessary to use only medical device measurement data measured by the medical device 11, the learning DB 253 is generated by extracting, of the measurement data stored in the integration DB 251, the medical device measurement data in the medical device 11 registered in the approved device DB 252.

Specifically, Device. A (Device version 2) manufactured by Manufacturer AA and Device. C (Device versions A and B) manufactured by Manufacturer BB are registered in the approved device DB 252 as the medical device 11. Therefore, the learning DB 253 is generated by extracting medical device measurement data including Device. A (Device version 2) and Device. C (Device version B) (by filtering the three records of the medical device 11) from the measurement data stored in the integration DB 251.

That is, in this example, old measurement data (measurement data at a time of Device version 1) in the measurement device (Device. A) certified as a medical device by a version upgrade of the measurement device and measurement data in the measurement device not certified as a medical device (Device. B) are excluded when the learning DB 253 is generated.

Furthermore, because it is possible to use medical device measurement data and non-medical device measurement data at a time of learning of a determination model for daily guideline, it is only required to generate the learning DB 253 by using all the measurement data stored in the integration DB 251.

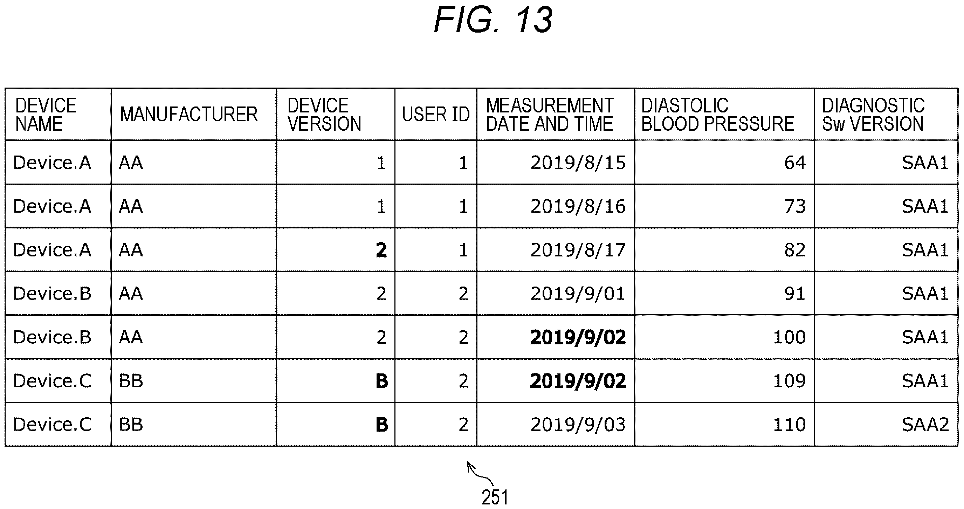

Fig. 13 illustrates another example of the integration DB 251.

In the table illustrated in Fig. 13, a column with a column name, which is DIAGNOSTIC Sw VERSION is added as compared with the table illustrated in Fig. 10.

Diagnostic Sw version stores information regarding a version of diagnostic software as software information.

Thus, by managing the diagnostic Sw version, it is possible to manage not only hardware of the measurement devices described above but also version information of software. For example, although it is assumed that the diagnostic software alone is treated as a medical device program or the like, a learning DB 253 corresponding to intended use can be generated by managing the diagnostic Sw version.

### (Configuration of server)

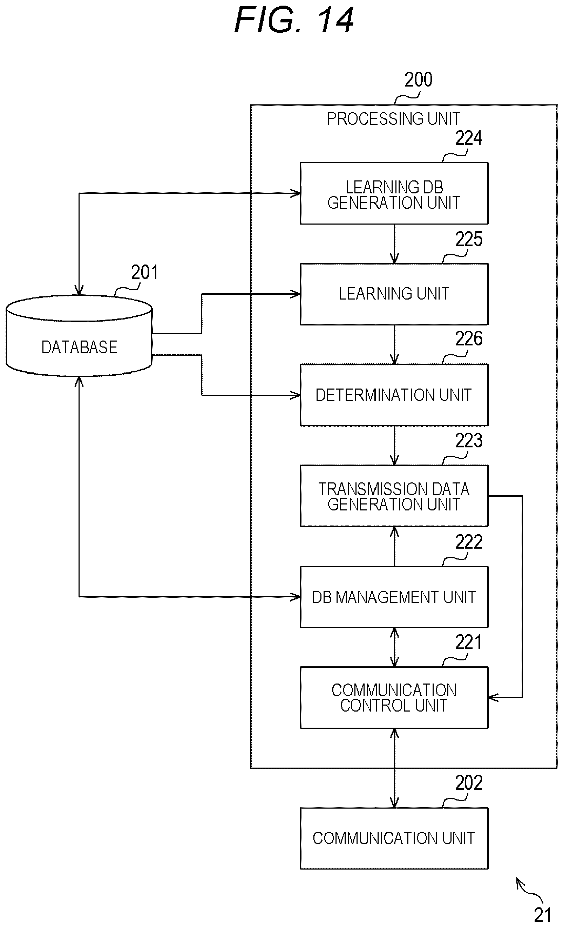

Fig. 14 illustrates another example of the configuration of the server 21 in Fig. 1 as a configuration of the server 21 that supports processing related to a determination model.

In Fig. 14, the parts corresponding to the configuration in Fig. 3 are provided with the same reference signs, and description thereof will be omitted as appropriate to avoid a repetition.

The processing unit 200 includes the communication control unit 221, the DB management unit 222, the transmission data generation unit 223, a learning DB generation unit 224, a learning unit 225, and a determination unit 226. Furthermore, the database 201 includes the integration DB 251, the approved device DB 252, and the learning DB 253.

The learning DB generation unit 224 generates a learning DB 253 corresponding to intended use by using the measurement data stored in the integration DB 251 on the basis of the information stored in the approved device DB 252. Note that the medical device determiner 261 (Fig. 9) is included in the learning DB generation unit 224.

The learning unit 225 learns with machine learning using the measurement data stored in the learning DB 253 corresponding to intended use so as to suit intended use, and generates a determination model for medical intervention or a determination model for daily guideline. The generated determination model (learned model) is supplied to the determination unit 226.

The determination unit 226 applies the determination model supplied from the learning unit 225 to the measurement data of the user as a determination target (measurement target), the measurement data being stored in the database 201. Note that it can be said that the user as the determination target here is a user as a measurement target of the measurement device. A result of determination with the determination model is supplied to the transmission data generation unit 223.

The transmission data generation unit 223 generates, on the basis of the result of the determination with the determination model supplied from the determination unit 226 and the measurement data supplied from the DB management unit 222, transmission data to be transmitted to the information device 13, and supplies the transmission data to the communication control unit 221.

### (Flow at time of learning)

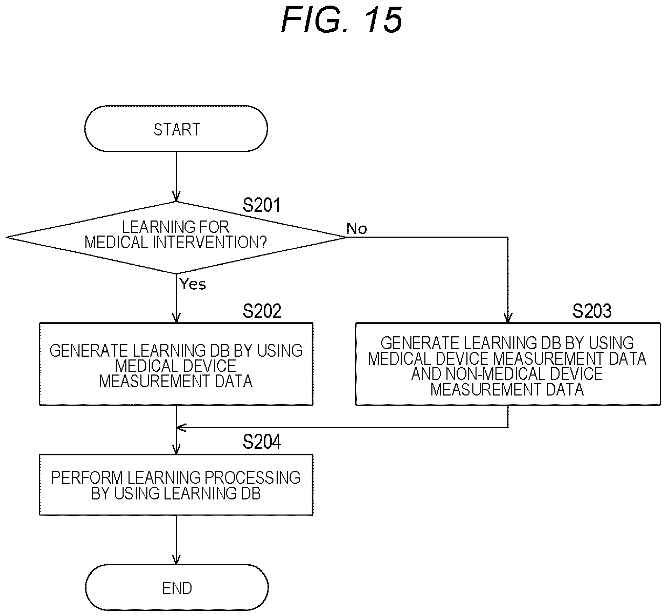

Next, a flow of processing at a time of learning will be described with reference to the flowchart in Fig. 15. The processing at a time of learning is executed by the processing unit 200 of the server 21.

In determination processing in Step S201, whether or not the learning is learning of the determination model for medical intervention is determined.

For example, by the physician operating a predetermined setting screen displayed on the information device 13 or the like to set intended use, a proration rate, or the like, generation of a determination model based on the intended use or the proration rate is instructed. Specifically, the intended use includes use for medical intervention, daily guideline, or the like. Furthermore, the proration rate includes weight of non-medical device measurement data or the like.

In a case where it is determined in the determination processing in Step S201 that the learning is learning of the determination model for medical intervention, the processing proceeds to Step S202.

In Step S202, the learning DB generation unit 224 generates the learning DB 253 by using the medical device measurement data. Specifically, the learning DB 253 is generated by extracting, from the measurement data stored in the integration DB 251, the medical device measurement data in the medical device 11 registered in the approved device DB 252.

Meanwhile, in a case where it is determined in the determination processing in Step S201 that the learning is not learning of the determination model for medical intervention, that is to say, the learning is learning by the determination model for daily guideline, the processing proceeds to Step S203.

In Step S203, the learning DB generation unit 224 generates the learning DB 253 by using the medical device measurement data and the non-medical device measurement data. Specifically, the learning DB 253 is generated by using the measurement data stored in the integration DB 251 without limitation.

When the processing in Step S202 or S203 ends, the processing proceeds to Step S204.

In Step S204, the learning unit 225 performs learning processing by using the generated learning DB 253.

Specifically, because a learning DB 253 in which medical device measurement data is stored is generated at a time of learning of the determination model for medical intervention, the learning is performed in the learning unit 225 so as to suit intended use (for medical intervention) with machine learning using the medical device measurement data stored in the learning DB 253. This learning generates a determination model for medical intervention corresponding to an instruction from the physician.

Furthermore, because a learning DB 253 in which measurement data in the medical device 11 and non-medical device 12 is stored is generated at a time of learning of the determination model for daily guideline, the learning is performed in the learning unit 225 so as to suit intended use (for daily guideline) with machine learning using the medical device measurement data and the non-medical device measurement data stored in the learning DB 253. This learning generates a determination model for daily guideline corresponding to an instruction from the physician.

The flow of processing at a time of learning has been described above. In the processing at a time of learning, the measurement data is not differentiated into the medical device measurement data and the non-medical device measurement data when collected from the measurement devices, and is differentiated into the medical device measurement data and the non-medical device measurement data at the time of learning to generate the learning DB 253. Then, by using this learning DB 253, a determination model corresponding to intended use is generated.

With this arrangement, even if Pharmaceutical and Medical Device Act will stipulate in a future that "For use in XX, non-medical devices (medical devices) may be used for up to YY% of a judgment" for example, a determination model compliant with the new stipulation can be generated by, corresponding to the stipulation, specifying an upper limit value of weight of non-medical device measurement data at a time of learning.

### (Flow at time of determination)

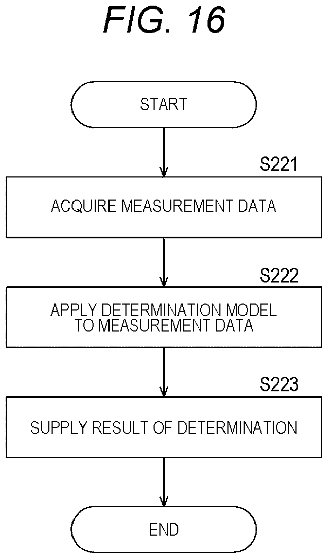

Next, a flow of processing at a time of determination will be described with reference to the flowchart in Fig. 16.

This determination processing may be executed not only by (a determination unit 206 of) the processing unit 200 of the server 21 as a matter of course, but also by the processing unit 100 of the information device 13. Here, description will be given assuming that the processing unit 200 executes the observation screen generation processing.

In Step S221, the determination unit 226 acquires the measurement data of the user as the determination target, the measurement data being stored in the database 201. For example, in a case where the physician who operates a predetermined setting screen displayed on the information device 13 or the like specifies the user (patient) as the determination target, measurement data measured by a measurement device used by the user is acquired.

In Step S222, the determination unit 226 applies the determination model generated in the above-described processing at the time of learning to the acquired measurement data.

Specifically, in a case where a medical intervention is determined, a determination result can be obtained by applying the measurement data (medical device measurement data) of the user as the determination target to the determination model for medical intervention. Furthermore, in a case where a daily guideline is determined, a determination result can be obtained by applying the measurement data (medical device measurement data and non-medical device measurement data) of the user as the determination target to the determination model for daily guideline.

The result of the determination with the determination model includes, for example, a disease estimation result of the user (patient) as the determination target or an action recommendation result for the user as the determination target.

In Step S223, the determination unit 226 supplies the result of the determination with the determination model to the transmission data generation unit 223.

The flow of processing at a time of determination has been described above. In the processing at a time of determination, a patient can be determined by using the determination model corresponding to intended use such as for medical intervention or for daily guideline, the determination model being generated in the processing at the time of learning. The result of the determination with the determination model can be included in the determination result screen.

### (Display example of determination result screen)

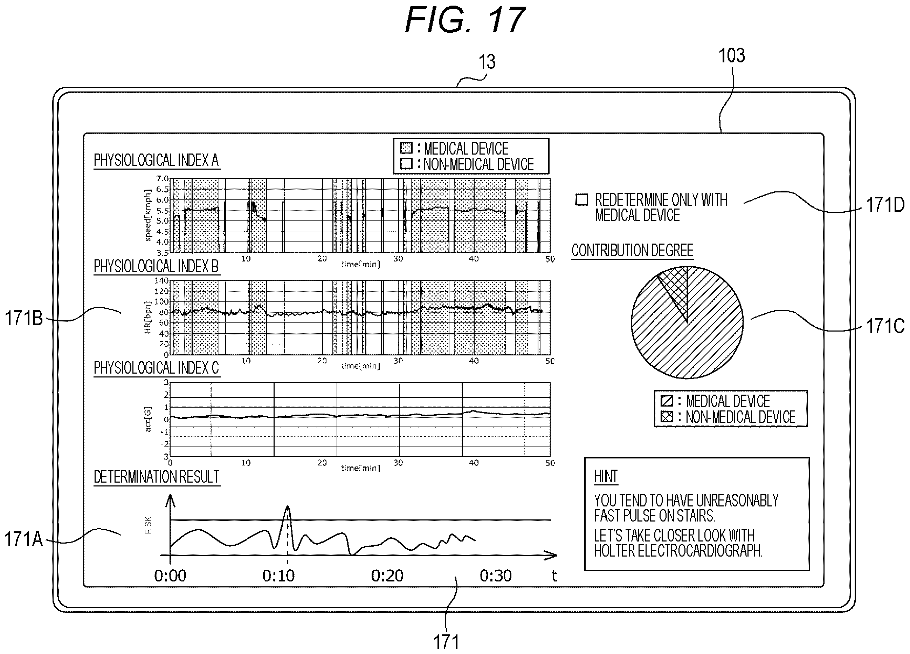

Fig. 17 illustrates an example of a determination result screen displayed on the display unit 103 of the information device 13 such as a tablet terminal used by the physician or the like.

In Fig. 17, a determination result screen 171 mainly includes four regions that are a determination result region 171A, a physiological index region 171B, a contribution degree region 171C, and a redetermination instruction region 171D.

The determination result region 171A displays a result of the determination with the determination model. In this example, as the result of the determination with the determination model, a graph illustrating a disease estimation result of the user as the determination target (risk) in time series and hint information representing an action recommendation result for the user are displayed. Note that Fig. 17 illustrates an example of a case where a determination model for daily guideline is used.

In this example, information that is "YOU TEND TO HAVE UNREASONABLY FAST PULSE ON STAIRS. LET'S TAKE CLOSER LOOK WITH HOLTER ELECTROCARDIOGRAM." is displayed as the hint information. Here, a time zone with a risk and a physiological index with a great contribution degree can be included. Furthermore, exemplification of the medical device 11 with respect to the physiological index, or the like can be performed.

The physiological index region 171B displays a measured value of a physiological index as a basis for determination on the result of the determination with the determination model. Physiological indices A to C are exemplified in this example, and body temperature, a pulse rate, behavior recognition (context), blood pressure, a respiratory rate, electrocardiogram, a blood glucose level, blood test value, maximum oxygen uptake (VO₂max), a lactate threshold (LT), a stress index (LF/HF), brain natriuretic peptide (BNP), or the like can be displayed as a physiological index.

The physiological indices A to C are displayed as a graph illustrating measurement data in a specified section in time series, or the like. When the measurement data is represented in time series in this graph, medical device measurement data measured by the medical device 11 and non-medical device measurement data measured by the non-medical device 12 can be displayed so as to be differentiated from each other by color or the like for each section.

The contribution degree region 171C displays contribution degrees of the medical devices 11. In this example, contribution degrees of the measurement devices are represented by a pie chart, and the contribution degree of the medical device 11 is about 90%. For example, a contribution degree can be calculated by regarding weight after learning as a contribution degree of a measurement device.

Although a description field in this pie chart displays that the hatched region represents the contribution degrees of the "medical devices" (about 90%), and the lattice pattern region represents the contribution degrees of the "non-medical devices" (about 10%), the regions may be displayed with specific device names or the like. Whether to display an abstract name such as "medical device" or "non-medical device" or to display a specific name such as "DEVICE NAME" can be selected by the physician or the like.

The redetermination instruction region 171D is a region for instructing execution of redetermination using only medical device measurement data measured by the medical device 11. This instruction region includes a button, a check box, or the like and redetermination is executed in a case where predetermined operation (tap operation or the like) is performed by the physician or the like.

Note that, for the redetermination instruction region 171D, an allowable proportion of the non-medical device 12 may be able to be specified similarly to the automatic arrangement button 151D in the observation screen 151 (Fig. 4), and redetermination corresponding to the specified allowable proportion may be performed. At this time, a section measured only by the non-medical device 12 is only required to be masked.

### (Flow of generation of determination result screen)

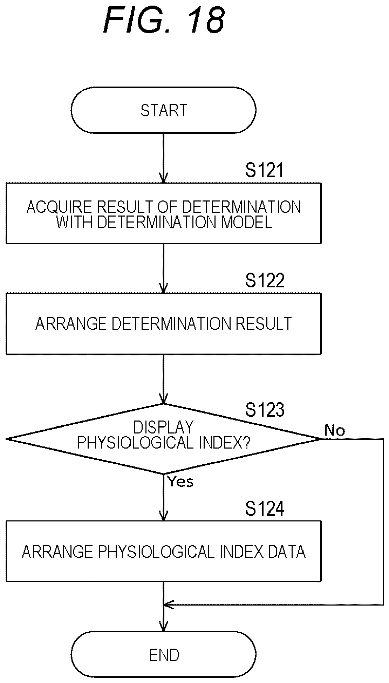

Next, a flow of determination result screen generation processing will be described with reference to the flowchart in Fig. 18.

The determination result screen generation processing is executed by the processing unit 100 (screen generation unit 122 or the like) of the information device 13, or is executed by the processing unit 200 (transmission data generation unit 223 or the like) of the server 21. Here, description will be given assuming that the processing unit 200 executes the observation screen generation processing.

In Step S121, the transmission data generation unit 223 acquires the result of the determination with the determination model supplied from the determination unit 226.

In Step S122, the transmission data generation unit 223 arranges the acquired determination result in a predetermined region. With this arrangement, the result of the determination with the determination model (disease estimation result, action recommendation result, or the like) is arranged in the determination result region 171A on the determination result screen 171 (Fig. 17),

In Step S123, the transmission data generation unit 223 determines whether or not to display the physiological index that is the basis for determination on the result of the determination with the determination model.

In a case where it is determined in the determination processing in Step S123 that the physiological index is to be displayed, the processing proceeds to Step S124. In Step S124, the transmission data generation unit 223 arranges physiological index data in a predetermined region. As the physiological index data, measurement data stored in the integration DB 251 can be used.

With this arrangement, a measured value of a physiological index such as body temperature or a pulse rate is arranged in the physiological index region 171B on the determination result screen 171.

Note that, in a case where it is determined in the determination processing in Step S123 that the physiological index is not to be displayed, Step S124 is skipped.

The determination result generated in this way and the transmission data including the physiological index are transmitted to the information device 13 via the network 31. Then, in the information device 13, the processing unit 100 displays the determination result screen 171 on the display unit 103 on the basis of the transmission data received from the server 21.

Note that, in a case where the processing unit 100 of the information device 13 executes the determination result screen generation processing, the server 21 is only required to transmit, as transmission data, the result of the determination with the determination model by the determination unit 226 and the measurement data stored in the database 201 to the information device 13 via the network 31.

The flow of the determination result screen generation processing has been described above. In the determination result screen generation processing, the determination result screen 171 using not only medical device measurement data but also non-medical device measurement data as measurement data and including the determination result and physiological index is generated, and therefore, the non-medical device measurement data can be effectively utilized. Furthermore, the physician can display the determination result screen 171 corresponding to intended use on the information device 13 and observe the determination result and the physiological index.

Here, a utilization example of the determination model for daily guideline basically assumes utilization out of a range of medical judgment. At that time, information corresponding to the determination result may be presented not only on the information device 13, such as a tablet terminal, used by the physician or the like, but also on the non-medical device 12 such as a wearable terminal worn by the user (patient).

For example, a consultation advice based on a result of the determination with the determination model for daily guideline, such as "YOU SHOULD HAVE XX EXAMINED AT HOSPITAL" or "DO YOU WANT TO GET APPOINTMENT FOR HOSPITAL?" can be displayed on the non-medical device 12 as an action recommendation.

Furthermore, in a case where detailed log information is required, the non-medical device 12 worn by the user may prompt measurement thereof. For example, when a behavior such as arrhythmia is sensed as a result of the determination with the determination model for daily guideline, the non-medical device 12 can display a message that prompts measurement such as "LET'S TAKE ELECTROCARDIOGRAM (ECG) TODAY, TOO." as an action recommendation.

Note that, in a case where the user contacts the physician and is diagnosed in detail when remembering that XX has hurt while checking log information, the action may be reflected in the log information.

### (Example of medical interview support tool for patient)

Incidentally, the present technology may be utilized by the patient to remember a symptom in the patient when the physician has a medical interview with the patient about a medical history or medical condition as a reference for diagnosis.

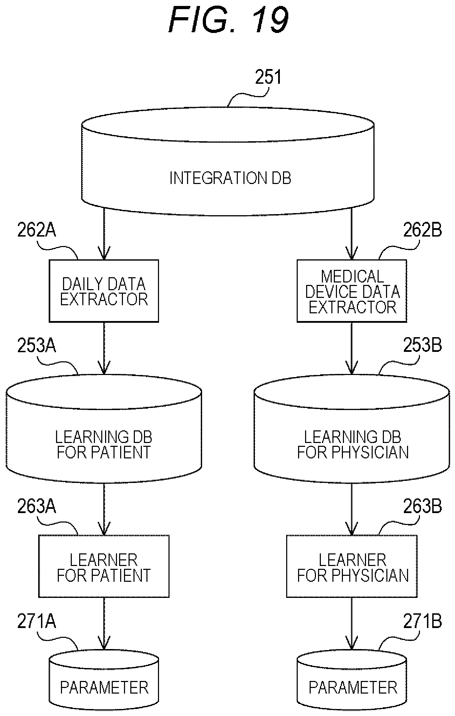

Fig. 19 illustrates a flow of processing at a time of learning.

In Fig. 19, a daily data extractor 262A extracts daily data from the measurement data stored in the integration DB 251. Daily data is data related to daily life of the user (patient), and is mainly non-medical device measurement data. A learning DB for patient 253A is generated by using the daily data.

A learner for patient 263A generates a determination model (learned model) learned with machine learning using daily data stored in the learning DB for patient 253A, and outputs a parameter 271A thereof. At this time, the learner for patient 263A can access to only daily data.

Furthermore, a medical device data extractor 262B extracts the medical device measurement data from the measurement data stored in the integration DB 251. A learning DB for physician 253B is generated by using the medical device measurement data.

A learner for physician 263B generates a determination model (learned model) learned with machine learning using the medical device measurement data stored in the learning DB for physician 253B, and outputs a parameter 271B thereof.

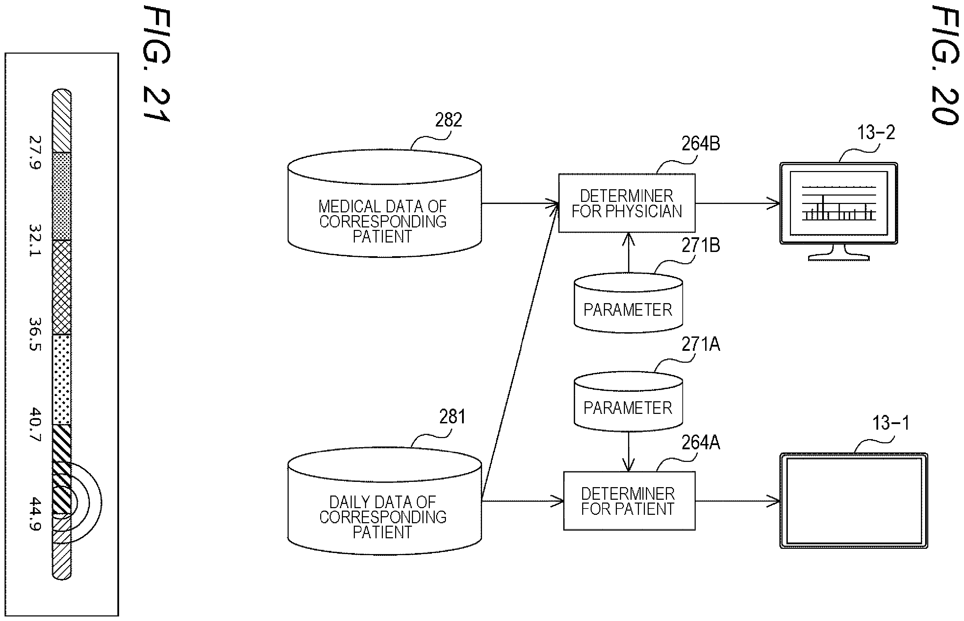

Fig. 20 illustrates a flow of processing at a time of determination.

In Fig. 20, the parameter 271A obtained at the time of learning is input to a determiner for patient 264A. On the basis of the input parameter 271A, the determiner for patient 264A makes a determination using the daily data of a corresponding patient, the daily data being read from a daily data DB 281. At this time, the determiner for patient 264A can access to only daily data. Note that the daily data includes non-medical device measurement data.

A result of determination by the determiner for patient 264A is displayed on the display unit 103 of an information device 13-1. With this arrangement, the patient can remember a symptom in the patient at a time of a medical interview by checking information such as the determination result displayed on the information device 13-1.

Furthermore, the parameter 271B obtained at the time of learning is input to a determiner for physician 264B. On the basis of the input parameter 271B, the determiner for physician 264B makes a determination using the daily data of the corresponding patient, the daily data being read from the daily data DB 281, and medical data of the corresponding patient, the medical data being read from a medical data DB 282. Note that the medical data includes medical device measurement data.

A result of determination by the determiner for physician 264B is displayed on the display unit 103 of an information device 13-2. With this arrangement, the physician can conduct a medical interview with or diagnose the corresponding patient while checking information such as the determination result displayed on the information device 13-2.

Thus, by utilizing a medical interview support tool for patient, the patient can check the result of the determination by the determiner for patient 264A at the time of the medical interview and remember the symptom in the patient. Furthermore, by changing the learning data at the time of learning or blocking an access by the patient to the medical data at the time of determination, it is possible to improve convenience of the medical interview and avoid unnecessary information leakage.

Moreover, it is possible to improve convenience at a time of checking a determination result, because a result of determination by the determiner for patient 264A and a result of determination by the determiner for physician 264B can be displayed separately on the information device 13-1 for the patient and the information device 13-2 for the physician.

Note that the configurations illustrated in Figs. 19 and 20 can be provided mainly as functions of the server 21. For example, the daily data extractor 262A and the medical device data extractor 262B are included in the learning DB generation unit 224, the learner for patient 263A and the learner for physician 263B are included in the learning unit 225, and the determiner for patient 264A and the determiner for physician 264B are included in the determination unit 226.

Furthermore, for example, the database 201 stores the integration DB 251, the learning DB for patient 253A and learning DB for physician 253B, the parameter 271A and parameter 271B, and the daily data DB 281 and medical data DB 282.

Furthermore, although a case where the daily data DB 281 and the medical data DB 282 are separately provided is exemplified in Figs. 19 and 20, the integration DB 251 may be used instead of the daily data DB 281 and the medical data DB 282.

### <2. Modifications>

### (Other display examples of screen)

Although, in the above description, the display examples of the observation screen 151 in Fig. 4 and the determination result screen 171 in Fig. 17 are illustrated as screens displayed on the information device 13 of the tablet terminal or the like, the screens may be displayed on a device other than the information device 13, and another display form may be used.

For example, in a case where the non-medical device 12, such as a wearable terminal, has a display unit, it is only required to perform the following to display, on a screen (a small-sized screen) of the display unit, information regarding the medical device 11 and information regarding the non-medical device 12 differentiated from each other.

That is to say, when the screen is displayed in the non-medical device 12, in order to differentiate information regarding the medical device 11 and information regarding the non-medical device 12, a display attribute of those pieces of information can be adjusted. Specifically, representation of a numerical value itself can be changed between the medical device 11 and the non-medical device 12 by adjusting an attribute such as color, line thickness, size, italic type, shadowed, or font of measured values.

Furthermore, the information regarding the medical device 11 and the information regarding the non-medical device 12 may be differentiated from each other by representation other than the representation of the numerical value itself, such as by displaying a still image, an icon, or the like before or in a region around a numerical value, such as the measured value, surrounding the numerical value with a frame, or underlining the numerical value.

Moreover, graphical representation, such as a graph changing in time series or a diagram illustrating a comparison with contemporaries, may be used. Fig. 21 illustrates an example of display in a case where a graphical representation is used for the non-medical device 12.

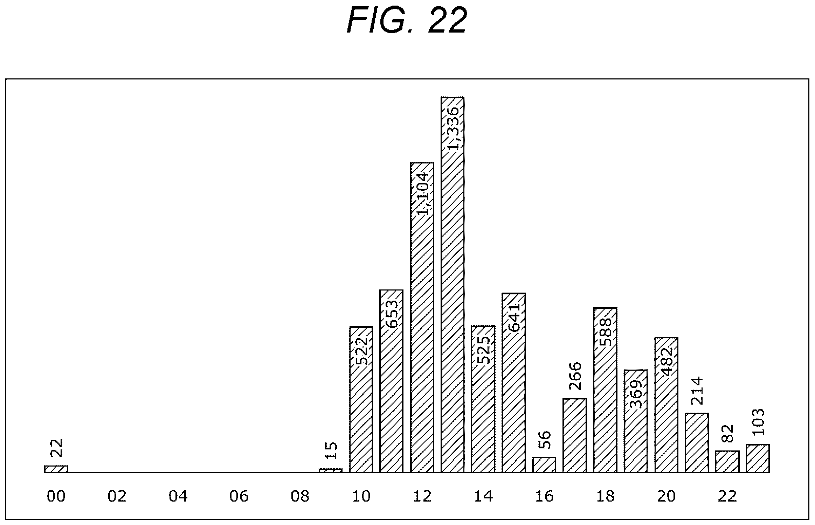

Furthermore, in a case where measured values changing in time series are represented with a bar chart for the non-medical device 12 as illustrated in Fig. 22, the information regarding the medical device 11 and the information regarding the non-medical device 12 may be differentiated from each other by not adding a numerical value to a value measured by the non-medical device 12 and adding a numerical value to a value measured by the medical device 11, or the like.

Note that, in order to differentiate the information regarding the medical device 11 and the information regarding the non-medical device 12 at a time of screen transition in the non-medical device 12, it is only required to use a fact that, for example, the number of times of transition until the information is displayed is different, different folders are used to store the information, or the like.

### (Another configuration example of system)

Although the above description focuses on a case where the observation screen 151 and the determination result screen 171 are displayed on the information device 13 of the information processing system 1, the screens may be displayed on the medical device 11 or the non-medical device 12, and further, the medical device 11 or the non-medical device 12 may be configured as the information device 13.

Furthermore, although the above description focuses on a case where processing of determination and processing of screen generation are performed in the server 21 of the information processing system 1 by using a determination model, the pieces of processing may be performed in the information device 13 or the like. That is to say, the processing of determination and processing of screen generation using a determination model may be executed either in (the processing unit 200 of) the server 21 on a cloud side or in (the processing unit 100 of) the information device 13 on a local side, or the like.

Furthermore, the server 21 has the processing unit 200 and the database 201 in the above description, the processing unit 200 and the database 201 may be separately provided in different servers. Moreover, the database 201 includes the integration DB 251, the approved device DB 252, and the learning DB 253 in the above description, each of the databases may be separately managed in different servers.

Moreover, although a case where a result of determination with a determination model is displayed on various screens is described above, for example, a method for presenting the result of the determination with the determination model is not limited to display, and the determination result may be presented by using another presentation method, such as voice reading of an action recommendation result.

Note that, in the present description, a learner corresponds to a learning model, a learner capable of obtaining sufficient accuracy after learning by using learning data is a learned model (determination model), and a determiner corresponds to the determination model. Furthermore, in the above description, the "user" basically means a "patient".

### <3. Configuration of computer>

A series of processing (for example, observation screen generation processing, determination result screen generation processing, processing at a time of learning, processing at a time of determination, or the like) executed by the information device 13 or server 21 described above can be executed by hardware or executed by software. In a case where a series of processing is executed by software, a program included in the software is installed on a computer in each apparatus.

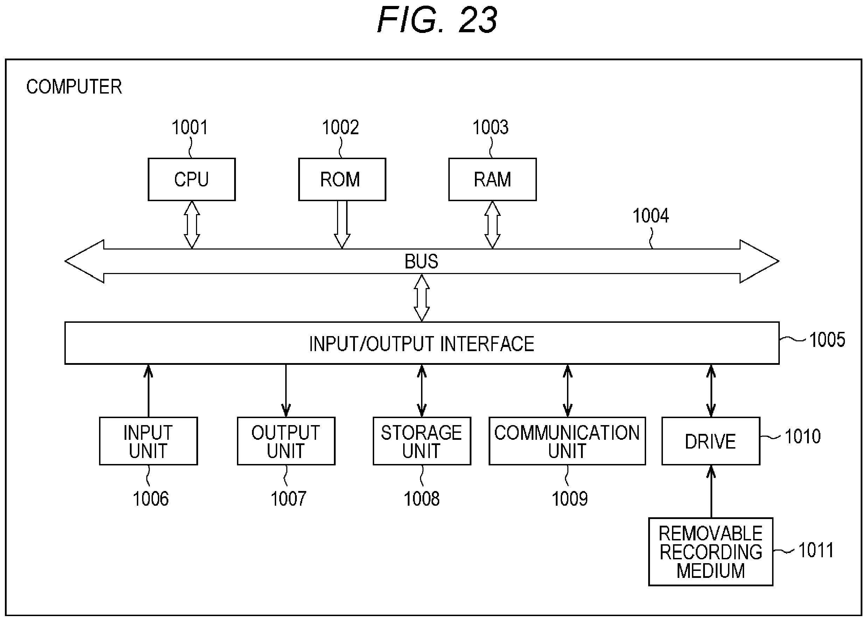

Fig. 23 is a block diagram illustrating a configuration example of hardware of a computer that executes the series of processing described above by a program.

In the computer, a central processing unit (CPU) 1001, a read only memory (ROM) 1002, and a random access memory (RAM) 1003 are mutually connected by a bus 1004. Moreover, an input/output interface 1005 is connected to the bus 1004. An input unit 1006, an output unit 1007, a storage unit 1008, a communication unit 1009, and a drive 1010 are connected to the input/output interface 1005.

The input unit 1006 includes a microphone, a keyboard, a mouse, or the like. The output unit 1007 includes a speaker, a display, or the like. The storage unit 1008 includes a hard disk, a non-volatile memory, or the like. The communication unit 1009 includes a network interface, or the like. The drive 1010 drives a removable recording medium 1011 such as a magnetic disk, an optical disk, a magnetooptical disk, or a semiconductor memory.

In the computer configured as above, the series of processing described above is executed by the CPU 1001 loading a program recorded in the ROM1002 or storage unit 1008 to the RAM 1003 via the input/output interface 1005 and the bus 1004 and executing the program.

A program executed by the computer (CPU 1001) can be provided by being recorded on the removable recording medium 1011 as a package medium, or the like, for example. Furthermore, the program can be provided via a wired or wireless transmission medium such as a local area network, the Internet, or digital satellite broadcasting.

In the computer, the program can be installed on the storage unit 1008 via the input/output interface 1005 by attaching the removable recording medium 1011 to the drive 1010. Furthermore, the program can be received by the communication unit 1009 via the wired or wireless transmission medium and installed on the storage unit 1008. In addition, the program can be installed on the ROM 1002 or the storage unit 1008 in advance.

Here, in the present description, processing performed by a computer according to the program does not necessarily have to be performed in time series in an order described as a flowchart. That is, processing performed by the computer according to a program also includes processing that is executed in parallel or individually (for example, parallel processing or object processing). Furthermore, the program may be processed by one computer (processor) or may be subjected to distributed processing by a plurality of computers.

Note that embodiments of the present technology are not limited to the above-described embodiments, and various changes can be made without departing from the gist of the present technology.

Furthermore, each step of a series of processing described above can be executed by one apparatus, or can be executed by being shared by a plurality of apparatuses. Moreover, in a case where a plurality of pieces of processing is included in one step, the plurality of pieces of processing included in the one step can be executed by being shared by a plurality of apparatuses, in addition to being executed by one apparatus.

Note that the following configurations can be used for the present technology.
(1) An information processing apparatus including
   a processing unit that makes a determination regarding a user as a measurement target according to intended use by using a determination model that has learned with machine learning using first measurement data measured by a medical device and second measurement data measured by a non-medical device so as to suit intended use.
(2) The information processing apparatus according to (1), in which, for the determination model, weight of the second measurement data is changed according to intended use.
(3) The information processing apparatus according to (2),
   in which the determination model is
   generated by machine learning using the first measurement data and the second measurement data in a case where the determination model is used for determination on a daily guideline, and
   generated by machine learning using the first measurement data in a case where the determination model is used for determination on medical intervention.
(4) The information processing apparatus according to any one of (1) to (3),
   in which the first measurement data and the second measurement data are obtained by differentiating collected measurement data at a time of learning.
(5) The information processing apparatus according to any one of (1) to (4),
   in which a result of determination using the determination model includes a disease estimation result of the user or an action recommendation result for the user.
(6) The information processing apparatus according to any one of (1) to (5),
   in which the processing unit outputs the result of the determination using the determination model.
(7) The information processing apparatus according to (4),
   in which the measurement data includes vital sign information regarding a vital sign detected by a vital sign sensor, and measurement device information regarding a measurement device.
(8) The information processing apparatus according to (7),
   in which the measurement device information includes a name of the measurement device, a manufacturer of the measurement device, and information regarding a version.
(9) The information processing apparatus according to (7) or (8),
   in which the vital sign information includes a value measured by the measurement device, time information regarding measurement, and identification information of the user as the measurement target.
(10) The information processing apparatus according to (6),
   in which the determination result is displayed in a predetermined display form on a determination result screen.
(11) The information processing apparatus according to (10),
   in which the determination result screen includes, along with the determination result, information regarding a physiological index as a basis for the determination result.
(12) The information processing apparatus according to (10) or (11),
   in which the determination result screen includes information regarding a contribution degree of the medical device.
(13) The information processing apparatus according to (1),
   in which the first measurement data and the second measurement data are displayed in a predetermined display form on an observation screen.
(14) The information processing apparatus according to (13),
   in which the observation screen includes information regarding an integration result in which information corresponding to the first measurement data or the second measurement data is arranged for each temporal section.
(15) An information processing method including,
   by an information processing apparatus,
   making a determination regarding a user as a measurement target according to intended use by using a determination model that has learned with machine learning using first measurement data measured by a medical device and second measurement data measured by a non-medical device so as to suit intended use.
(16) An information processing apparatus including
   a processing unit that learns, at a time of generation of a determination model for making a determination regarding a user as a measurement target according to intended use, with machine learning using first measurement data measured by a medical device and second measurement data measured by a non-medical device so as to suit intended use.
(17) The information processing apparatus according to (16),
   in which the processing unit generates the determination model by changing weight of the second measurement data according to intended use.
(18) The information processing apparatus according to (17),
   in which the processing unit
   performs machine learning using the first measurement data and the second measurement data in a case where the determination model used for determination on a daily guideline is generated, and
   performs machine learning using the first measurement data in a case where the determination model used for determination on medical intervention is generated.
(19) The information processing apparatus according to any one of (16) to (18),
   in which, at a time of learning, the processing unit differentiates the collected measurement data between the first measurement data and the second measurement data.
(20) An information processing method including,
   by an information processing apparatus,
   learning, at a time of generation of a determination model for making a determination regarding a user as a measurement target according to intended use, with machine learning using first measurement data measured by a medical device and second measurement data measured by a non-medical device so as to suit intended use.
(21) A program that causes a computer to function as
   a processing unit that learns, at a time of generation of a determination model for making a determination regarding a user as a measurement target according to intended use, with machine learning using first measurement data measured by a medical device and second measurement data measured by a non-medical device so as to suit intended use.

### REFERENCE SIGNS LIST

- 1: Information processing system
- 11, 11-1 to 11-N: Medical devices
- 12, 12-1 to 12-M: Non-medical devices
- 13: Information device
- 21: Server
- 31: Network
- 100: Processing unit
- 101: Input unit
- 102: Communication unit
- 103: Display unit
- 121: Communication control unit
- 122: Screen generation unit
- 123: Display control unit
- 200: Processing unit
- 201: Database
- 202: Communication unit
- 221: Communication control unit
- 222: DB management unit
- 223: Transmission data generation unit
- 224: Learning DB generation unit
- 225: Learning unit
- 226: Determination unit
- 251: Integration DB
- 252: Approved device DB
- 253: Learning DB
- 253A: Learning DB for patient
- 253B: Learning DB for physician
- 261: Medical device determiner
- 262A: Daily data extractor
- 262B: Medical device data extractor
- 263A: Learner for patient
- 263B: Learner for physician
- 264A: Determiner for patient
- 264B: Determiner for physician
- 281: Daily data of corresponding patient
- 282: Medical data of corresponding patient
- 1001: CPU

## Claims

1. An information processing apparatus comprising
a processing unit that makes a determination regarding a user as a measurement target according to intended use by using a determination model that has learned with machine learning using first measurement data measured by a medical device and second measurement data measured by a non-medical device so as to suit intended use.

2. The information processing apparatus according to claim 1,
wherein, for the determination model, weight of the second measurement data is changed according to intended use.

3. The information processing apparatus according to claim 2,
wherein the determination model is
generated by machine learning using the first measurement data and the second measurement data in a case where the determination model is used for determination on a daily guideline, and
generated by machine learning using the first measurement data in a case where the determination model is used for determination on medical intervention.

4. The information processing apparatus according to claim 1,
wherein the first measurement data and the second measurement data are obtained by differentiating collected measurement data at a time of learning.

5. The information processing apparatus according to claim 1,
wherein a result of determination using the determination model includes a disease estimation result of the user or an action recommendation result for the user.

6. The information processing apparatus according to claim 1,
wherein the processing unit outputs the result of the determination using the determination model.

7. The information processing apparatus according to claim 4,
wherein the measurement data includes vital sign information regarding a vital sign detected by a vital sign sensor, and measurement device information regarding a measurement device.

8. The information processing apparatus according to claim 7,
wherein the measurement device information includes a name of the measurement device, a manufacturer of the measurement device, and information regarding a version.

9. The information processing apparatus according to claim 7,
wherein the vital sign information includes a value measured by the measurement device, time information regarding measurement, and identification information of the user as the measurement target.

10. The information processing apparatus according to claim 6,
wherein the determination result is displayed in a predetermined display form on a determination result screen.

11. The information processing apparatus according to claim 10,
wherein the determination result screen includes, along with the determination result, information regarding a physiological index as a basis for the determination result.

12. The information processing apparatus according to claim 11,
wherein the determination result screen includes information regarding a contribution degree of the medical device.

13. The information processing apparatus according to claim 1,
wherein the first measurement data and the second measurement data are displayed in a predetermined display form on an observation screen.

14. The information processing apparatus according to claim 13,
wherein the observation screen includes information regarding an integration result in which information corresponding to the first measurement data or the second measurement data is arranged for each temporal section.

15. An information processing method comprising,
by an information processing apparatus,
making a determination regarding a user as a measurement target according to intended use by using a determination model that has learned with machine learning using first measurement data measured by a medical device and second measurement data measured by a non-medical device so as to suit intended use.

16. An information processing apparatus comprising
a processing unit that learns, at a time of generation of a determination model for making a determination regarding a user as a measurement target according to intended use, with machine learning using first measurement data measured by a medical device and second measurement data measured by a non-medical device so as to suit intended use.

17. The information processing apparatus according to claim 16,
wherein the processing unit generates the determination model by changing weight of the second measurement data according to intended use.

18. The information processing apparatus according to claim 17,
wherein the processing unit
performs machine learning using the first measurement data and the second measurement data in a case where the determination model used for determination on a daily guideline is generated, and
performs machine learning using the first measurement data in a case where the determination model used for determination on medical intervention is generated.

19. An information processing method comprising,
by an information processing apparatus,
learning, at a time of generation of a determination model for making a determination regarding a user as a measurement target according to intended use, with machine learning using first measurement data measured by a medical device and second measurement data measured by a non-medical device so as to suit intended use.

20. A program that causes a computer to function as
a processing unit that learns, at a time of generation of a determination model for making a determination regarding a user as a measurement target according to intended use, with machine learning using first measurement data measured by a medical device and second measurement data measured by a non-medical device so as to suit intended use.
